# EUROPEAN PATENT APPLICATION

(11) **EP 4 708 321 A2**
(43) Date of publication of application: **11.03.2026**
(21) Application number: 26152635.4
(22) Date of filing: 19.10.2023
(51) Int. Cl.: G16H 50/20

(54) **SYSTEMS FOR ADJUSTING A NEUROMODULATION THERAPY BASED ON PHYSIOLOGICAL INPUTS**

(30) Priority: 25.10.2022 US 202263419163 P; 17.10.2023 US 202318381003
(62) Divisional of application: 23204661.5
(71) Applicant: Medtronic, Inc., Minneapolis, Minnesota 55432 (US)
(72) Inventor: Straka, Malgorzata Maria, Blaine (US); Brinda, Annemarie K., Minneapolis (US); Hincapie, Juan G., Minneapolis (US); Litvak, Leonid M., Los Angeles (US); Mueller, Jerel Keith, Minneapolis (US); Nedrud, Joshua James, Minneapolis (US); Cleland, Andrew Jay, Minneapolis (US); Kharam, Aleksandra Pavlovna, Minneapolis (US); Sharan, Ashwini, Pennington (US)
(74) Representative: Zimmermann & Partner Patentanwälte mbB

(57) **Abstract**

A system for tracking one or more physiological-based inputs of a user, the system comprising:
a processor; and a memory storing data for processing by the processor, the data, when processed, causes the processor to: receive one or more physiological-based inputs at a first time, track the one or more physiological-based inputs after the first time and over a period of time to yield one or more changed physiological-based inputs, and determine one or more physiological responses based on a difference between the one or more physiological-based inputs received at the first time and the one or more changed physiological-based inputs; and determine if a physiological event has occurred based on the one or more physiological responses.

## Description

### CROSS-REFERENCE TO RELATED APPLICATIONS

This application claims the benefit of U.S. Provisional Application No. 63/419,163, filed on October 25, 2022, which application is incorporated herein by reference in its entirety.

### BACKGROUND

The present disclosure is directed to neuromodulation therapy and relates more particularly to adjusting and/or tracking a neuromodulation therapy based on one or more inputs and/or alerting a user based on the one or more inputs.

Open-loop or closed-loop neuromodulation therapy may be carried out by sending an electrical signal generated by a pulse generator to a stimulation target (e.g., nerves, non-neuronal cells, etc.), which may provide a desired electrophysiological, biochemical, or genetic response in the stimulation target. In closed-loop neuromodulation therapies, one or more signals resulting from the neuromodulation may be recorded and the therapy may be adjusted (whether automatically or manually) based on the recorded signals.

### BRIEF SUMMARY

Example aspects of the present disclosure include:
A system for generating a neuromodulation therapy of a user according to at least one embodiment of the present disclosure comprises a processor; and a memory storing data for processing by the processor, the data, when processed, causes the processor to: receive one or more physiological-based inputs of the user, determine a therapy response score based on the one or more physiological-based inputs, and generate at least one adjustment for one or more parameters of the neuromodulation therapy based on the therapy response score.

Any of the aspects herein, wherein the memory stores further data for processing by the processor that, when processed, causes the processor to: apply the at least one adjustment to the one or more parameters, wherein the one or more parameters are adjusted automatically or manually.

Any of the aspects herein, wherein the memory stores further data for processing by the processor that, when processed, causes the processor to: receive information about a state of a therapy system configured to deliver the neuromodulation therapy; and generate a notification based on at least one of the state of the therapy system, the therapy response score, or the one or more physiological-based inputs.

Any of the aspects herein, wherein the one or more parameters comprises at least one of increasing or decreasing a stimulation amplitude of a signal, increasing or decreasing a pulse width of a signal, increasing or decreasing a frequency of a signal, increasing or decreasing a number of electrode contacts, changing the electrode contacts that provide stimulation, starting or stopping the therapy, increasing or decreasing a cycling of the therapy, increasing or decreasing the cycling of high frequency components of the therapy independently of low frequency components of the therapy, adjustment of charge balancing strategy or recharge settings, or changing an on/off cycling time while maintaining a cycling ratio.

Any of the aspects herein, wherein the memory stores further data for processing by the processor that, when processed, causes the processor to receive one or more additional inputs and the therapy response score may be further based on the one or more additional inputs, wherein the one or more additional inputs comprises at least one of user feedback or an environmental signal.

Any of the aspects herein, wherein the one or more physiological-based inputs comprises at least one of an activity or accelerometer signal, a cardiac-derived metric, a sleep score, CMAP, EMG, respiration, an eCAP signal, posture, and/or other autonomic measures including organ sensing respiration.

Any of the aspects herein, wherein the one or more physiological-based inputs are received from at least one of a wearable device, a user device, or a neuromodulation therapy system.

Any of the aspects herein, wherein the one or more physiological-based inputs are received over a period of time and comprises one or more cardiac signals, and wherein the period of time is determined based on the one or more cardiac signals and input about the user's state.

Any of the aspects herein, wherein the memory stores further data for processing by the processor that, when processed, causes the processor to map the one or more changed physiological-based inputs to one or more events.

Any of the aspects herein, wherein the memory stores further data for processing by the processor that, when processed, causes the processor to generate a notification when at least one of the one or more events or the one or more physiological-based inputs are determined to be a risk to the user.

A system for tracking one or more physiological-based inputs of a user according to at least one embodiment of the present disclosure comprises a processor; and a memory storing data for processing by the processor, the data, when processed, causes the processor to: receive one or more physiological-based inputs at a first time, track the one or more physiological-based inputs after the first time and over a period of time to yield one or more changed physiological-based inputs, and determine one or more physiological responses based on a difference between the one or more physiological-based inputs received at the first time and the one or more changed physiological-based inputs; and determine if a physiological event has occurred based on the one or more physiological responses.

Any of the aspects herein, wherein the physiological event comprises a lead migration.

Any of the aspects herein, further comprising: a pulse generator configured to generate an electrical signal; and a lead in communication with the pulse generator and configured to transmit the electrical signal to an anatomical element.

Any of the aspects herein, wherein the tracking the one or more physiological-based inputs are tracked with at least one of stimulation or without stimulation.

Any of the aspects herein, wherein the tracking is repeated over two or more days for a similar period of time each day.

Any of the aspects herein, wherein the period of time comprises a first period of time and a second period of time
A system for adjusting a neuromodulation therapy of a user according to at least one embodiment of the present disclosure comprises a processor; and a memory storing data for processing by the processor, the data, when processed, causes the processor to: track one or more physiological-based inputs at a first time; track the one or more physiological-based inputs after the first time and over a period of time to yield one or more changed physiological-based inputs; and adjust one or more parameters of the neuromodulation therapy based on a difference between the one or more physiological-based inputs and the one or more changed physiological-based inputs.

Any of the aspects herein, wherein the period of time comprises a first period of time and a second period of time, the first period of time corresponding to when the user is awake and the second period of time corresponding to when the user is asleep.

Any of the aspects herein, wherein the period of time comprises a first period of time and a second period of time, the first period of time corresponding to when the user is active and the second period of time corresponding to when the user is inactive.

Any of the aspects herein, wherein the one or more physiological-based inputs comprises one or more cardiac signals, and wherein the period of time is determined based on the one or more cardiac signals and input about the user's state.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

Further disclosed herein are systems and methods for generating and/or applying an adjustment for one or more parameters of a neuromodulation therapy of a user, wherein one or more physiological-based inputs of the user may be received and a therapy response score may be determined based on the one or more physiological-based inputs, and wherein at least one adjustment for one or more parameters of the neuromodulation therapy may be generated based on the therapy response score.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is an illustration of an example neuromodulation therapy system according to at least one embodiment of the present disclosure;
Fig. 2 is an illustration of an example neuromodulation therapy system according to at least one embodiment of the present disclosure;
Fig. 3 is a block diagram of a system according to at least one embodiment of the present disclosure;
Fig. 4A is a flowchart according to at least one embodiment of the present disclosure;
Fig. 4B is a flowchart according to at least one embodiment of the present disclosure;
Fig. 5 is a flowchart according to at least one embodiment of the present disclosure;
Fig. 6 is a flowchart according to at least one embodiment of the present disclosure;
Fig. 7 is a flowchart according to at least one embodiment of the present disclosure; and
Fig. 8 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Cortex MX; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Neuromodulation therapy systems are conventionally used to deliver electrical stimulation to a stimulation target to provide a desired electrophysiologic, biochemical, or genetic response in the stimulation target. Common concerns of neuromodulation therapy systems include maintaining and customizing an effective neuromodulation therapy for an individualized user. To aid in customizing the neuromodulation therapy, physiological recordings from the neuromodulation therapy system may provide key information about a health state of the user, which can be used to adjust the neuromodulation therapy automatically (by, for example, a controller or computing device) or manually (by, for example, a user such as a medical provider, patient, or clinician). Notably, cardiac electrogram signals of high quality can be recorded from implanted leads of the neuromodulation therapy system. The cardiac electrograms and derived metrics can be combined with sensor output such as sensor data from an accelerometer to enable customization and adjustments to the neuromodulation therapy for a specific user and can be used to also detect certain risks relevant to the user.

Further, cardiac-derived measures, including heart rate and heart rate variability, may be useful biomarkers for chronic pain and indicators that the neuromodulation therapy is successful. Typically, these metrics are taken while the patient is at rest as a baseline metric. As such, embodiments of the present disclosure provide for measuring these metrics when the user is in different positions, during rest, and/or during user movement.

Other concerns include maintaining effective therapy while minimizing power consumption of the system to improve battery longevity and decreasing recharge burdens for rechargeable devices. According to at least one embodiment of the present disclosure, minimizing power consumption may include cycling the therapy on and off for specific periods of time, which has shown in some patients to maintain therapy efficacy and decrease power consumption. The cycling of therapy ON or OFF can be changed by increasing the ratio of cycling by maintaining the ON time while increasing the OFF time (or vice versa) or by changing the ON and OFF times but maintaining the ratio of therapy. Oftentimes, calibrating a user's optimal cycling is a time consuming, manual process. Thus, systems according to at least one embodiment of the present disclosures uses physiological-based inputs (and potentially user input and/or environmental inputs) to tailor the optimal cycling time and other parameters of the therapy to a user's needs over time, especially as the user's pain may change due to environmental factors or, in some instances, the progression of a disease state.

Thus, embodiments of the present disclosure provide technical solutions to one or more of the problems of (1) customizing an open-loop or closed-loop neuromodulation therapy for a user, (2) determining if lead(s) of a neuromodulation therapy system have migrated from an initial position within the user, (3) determining one or more health-based risks to the user using input(s) received from the neuromodulation therapy system, sensors, and/or the user, (4) modulating power consumption of the neuromodulation therapy system to maintain a quality of the neuromodulation therapy, and (5) increasing patient comfort and safety.

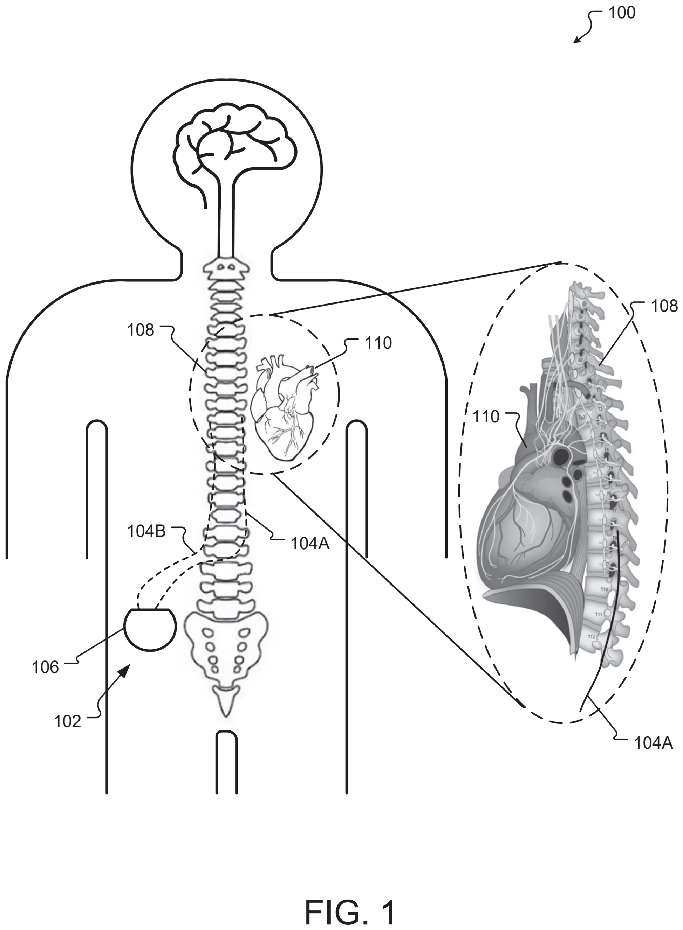

Turning to Fig. 1, a diagram of aspects of a neuromodulation therapy system 100 according to at least one embodiment of the present disclosure is shown. The neuromodulation therapy system 100 may be used to provide electric signals for a patient and/or carry out one or more other aspects of one or more of the methods disclosed herein. The neuromodulation therapy system 100 may be open-loop or closed-loop. In some examples, the device 102 may be referred to as a pulse generator. The pulse generator may be implantable in a patient or may be external to the patient. Additionally, the neuromodulation therapy system 100 may include one or more wires or leads 104 that provide a connection between the device 102 and nerves of the patient for enabling the stimulation/blocking therapy.

Neuromodulation techniques (e.g., technologies that act directly upon nerves of a patient, such as the alteration, or "modulation," of nerve activity by delivering electrical impulses or pharmaceutical agents directly to a target area) may be used to provide a desired electrophysiological, biochemical, or genetic response in the stimulation target for assisting in treatments for different diseases, disorders, or ailments of a patient, such as chronic pain. Accordingly, as described herein, the neuromodulation techniques may be used for altering the pain signals in the nervous system. For example, the device 102 may provide electrical stimulation to one or more nerves in the spinal cord of the patient (e.g., via the one or more leads 104) to block the pain signals from being received by the brain. In other examples, the device 102 may provide electrical stimulation to the brain. In still other embodiments, the electrical stimulation may be provided to, for example, ventral placed leads, leads placed on the dorsal root ganglion or the dorsal root entry zone, and/or any intervening regions around the spinal cord to optimize signal acquisition. In further embodiments, the electrical stimulation may be provided to leads place anywhere in the patient. It will be appreciated that the system 100 may include one device 102, two devices 102, or more than two devices 102.

In some examples, as shown in Fig. 1, the one or more leads 104 may include one lead 104. In other embodiments, the one or more leads 104 may include multiple leads such as a first lead 102A and a second lead 102B. In such embodiments, some of the leads 104 may provide active stimulation and other leads 104 may record a physiological response to the stimulation. It will be appreciated that in some embodiments that the lead 104 may both stimulate and record (e.g., some electrodes may provide the stimulation and other electrodes on the same lead may record the physiological response or the same electrodes may provide stimulation and recording). The lead 104 may be implanted on or near any anatomical element targeted for therapy. In some examples, the lead 104 is implanted near the spinal cord and more specifically, in the epidural space between the spinal cord and the vertebrae. Once implanted, the lead 104 may provide an electrical signal (whether stimulating or blocking) from the device 102 to the anatomical element (e.g., one or more nerves in the spinal cord, the brain, etc.). The device 102 in some embodiments, may be implanted in the patient, though in other embodiments the device 102 may be external to the patient's body.

In some examples, the lead 104 may provide the electrical signals to the respective nerves via electrodes that are connected to the nerves (e.g., sutured in place, wrapped around the nerves, etc.). In some examples, the lead 104 may be referenced as cuff electrodes or may otherwise include the cuff electrodes (e.g., at an end of the lead 104 not connected or plugged into the device 102). Additionally or alternatively, while shown as physical wires that provide the connection between the device 102 and the one or more nerves, the electrodes may provide the electrical signals to the one or more nerves wirelessly (e.g., with or without the device 102). In still other embodiments, it will be appreciated that the electrodes may not be positioned on the lead 104. For example, the electrodes may be on the device 102.

Additionally, the one or more leads 104 may be connected, placed, or otherwise implanted near or on a spinal cord 108 within the patient, such that at least one of the one or more leads 104 are located near a heart 110 of the patient. For example, as shown in the inset or zoomed-in portion of the example of Fig. 1 which depicts a side view of the encircled area within the patient, the first lead 104A may be placed within the spinal canal (for example, behind the foramen of the spine near the top of a vertebra of the thoracic vertebrae column) of the spinal cord 108 (e.g., T8 vertebrae) behind the heart 110. Additionally or alternatively, as described previously, the exact placement of the one or more leads 104 may vary depending on, for example, the type of treatment, the type of lead, the patient, combinations thereof, and the like. While not specifically shown in the example of Fig. 1, the one or more leads 104 may also exit the spinal cord 108 at a lumbar vertebra lower down the spinal cord 108 (e.g., the L2 vertebra, but the exact location may vary). As described herein, the one or more leads 104 being placed proximate to the heart 110 may enable the system 100 to more effectively capture signals that include cardiac activity before, during, and/or after providing a neuromodulation therapy (e.g., SCS therapy).

Additionally, while not shown, the neuromodulation therapy system 100 may include one or more processors (e.g., one or more DSPs, general purpose microprocessors, graphics processing units, ASICs, FPGAs, or other equivalent integrated or discrete logic circuitry) that are programmed to carry out one or more aspects of the present disclosure. In some examples, the one or more processors may be used to control one or more parameters of the neuromodulation therapy system 100. In other examples, the one or more processors may include a memory or may be otherwise configured to perform the aspects of the present disclosure. For example, the one or more processors may provide instructions to the device 102, the electrodes, or other components of the neuromodulation therapy system 100 not explicitly shown or described with reference to Fig. 1 for providing the pain blocking therapy to regulate chronic pain in a patient as described herein. In some examples, the one or more processors may be part of the device 102 or part of a control unit for the neuromodulation therapy system 100 (e.g., where the control unit is in communication with the device 102 and/or other components of the neuromodulation therapy system 100).

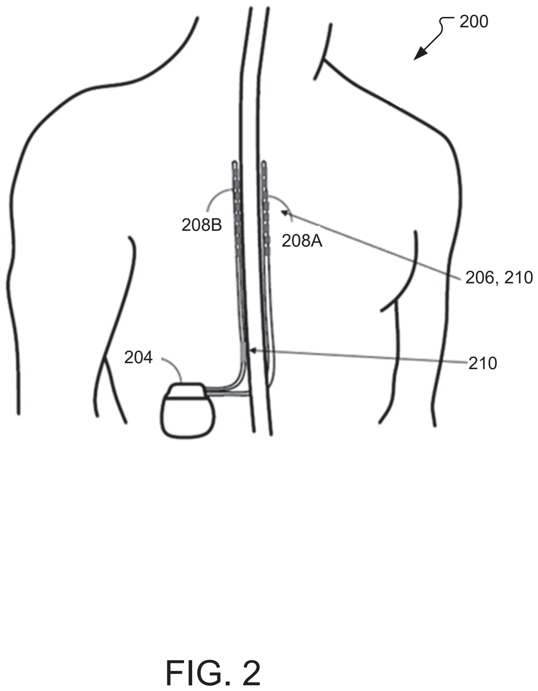

Turning to Fig. 2, an illustration of a neuromodulation therapy system 200 according to at least one embodiment of the present disclosure is shown. The neuromodulation therapy system 200 is the same as or similar to the neuromodulation therapy system 100 and comprises a device 204 which may be the same as or similar to the device 102. The neuromodulation therapy system 200 also includes a lead that comprises a first lead 208A and a second lead 208B. In some embodiments the neuromodulation therapy system 200 may be open-loop or closed-loop. The first lead 208A may comprise both stimulating electrodes 206 configured to stimulate a target anatomical element (e.g., a nerve) and recording electrodes 210 near or adjacent to the stimulating electrodes 206 and configured to record a physiological response to the stimulation (e.g., eCAP signals, cardiac signals, compound muscle action potential (CMAP), electromyography (EMG), respiration, etc.), posture, and/or other autonomic measures including organ sensing respiration. In some instances, the physiological response may be used to adjust (whether manually or automatically) one or more parameters of the neuromodulation therapy system 200. The second lead 208B may comprise both stimulating electrodes 206 configured to stimulate the target anatomical element and recording electrodes 210 positioned at a distance from the stimulating electrodes 206 and configured to record a physiological response to the stimulation. It will be appreciated that the second lead 208B may also include recording electrodes 210 positioned near or adjacent to the stimulating electrodes 206. The first lead 208A and the second lead 208B may be implanted near each other in in the same space (for example, the epidural space), or may be implanted in separate spaces. The device 204 may also contain additional electrodes or sensors for sensing physiological features.

The neuromodulation therapy system 100, 200 or similar systems may be used, for example, to carry out one or more aspects of any of the methods 500, 600, 700 and/or 800 described below. In some embodiments, the neuromodulation therapy system 100, 200 may be used with a system 300 for programming the lead 104 based on physiological-based inputs or results from a user. The neuromodulation therapy system 100, 200 or similar systems may also be used for other purposes. It will be appreciated that the human body has many nerves and the stimulation or neuromodulation therapies described herein may be applied to any one or more nerves, which may reside at any location of a patient (e.g., lumbar, thoracic, extremity, brain, trunk etc.). The stimulation or neuromodulation therapies may also be applied to any stimulation target (e.g., an anatomical element, non-neuronal cells, etc.)

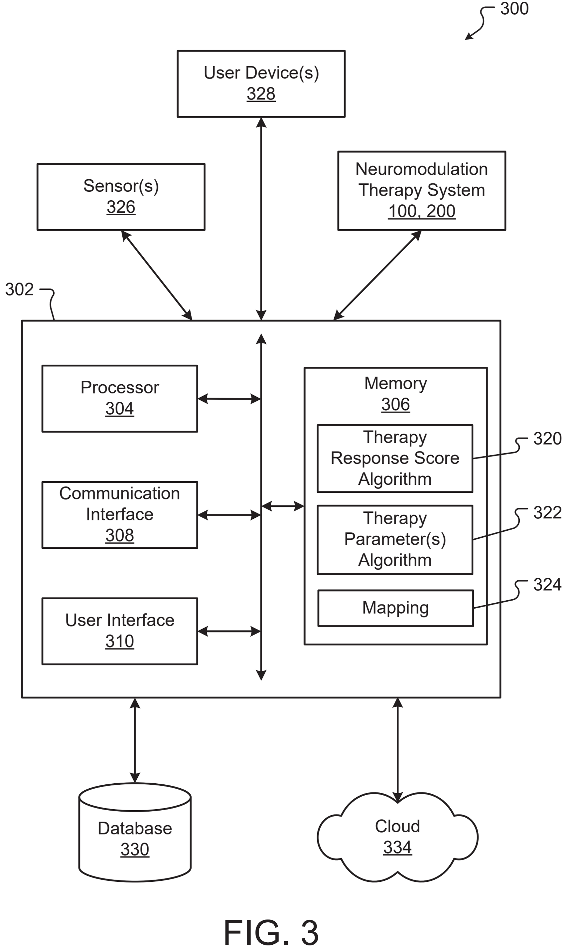

Turning to Fig. 3, a block diagram of a system 300 according to at least one embodiment of the present disclosure is shown. The system 300 may be used to for controlling the neuromodulation therapy system 100, 200 for programing one or more leads 104 of the neuromodulation therapy system 100, 200, and/or to carry out one or more other aspects of one or more of the methods disclosed herein. The system 300 comprises a computing device 302, the neuromodulation therapy system 100, 200, one or more sensor(s) 326, one or more wearable device(s) 328, a database 330, and/or a cloud or other network 334. Systems according to other embodiments of the present disclosure may comprise more or fewer components than the system 300. For example, the system 300 may not include the sensor 326, the user device 328, one or more components of the computing device 302, the database 330, and/or the cloud 334.

The computing device 302 comprises a processor 304, a memory 306, a communication interface 308, and a user interface 310. Computing devices according to other embodiments of the present disclosure may comprise more or fewer components than the computing device 302.

The processor 304 of the computing device 302 may be any processor described herein or any similar processor. The processor 304 may be configured to execute instructions stored in the memory 306, which instructions may cause the processor 304 to carry out one or more computing steps utilizing or based on data received from the sensor 326, the user device 328, the neuromodulation therapy system 100, 200, the database 330, and/or the cloud 334.

The memory 306 may be or comprise RAM, DRAM, SDRAM, other solid-state memory, any memory described herein, or any other tangible, non-transitory memory for storing computer-readable data and/or instructions. The memory 306 may store information or data useful for completing, for example, any step of the methods 500, 600, 700, and/or 800 described herein, or of any other methods. The memory 306 may store, for example, instructions and/or machine learning models that support one or more functions of the neuromodulation therapy system 100, 200. For instance, the memory 306 may store content (e.g., instructions and/or machine learning models) that, when executed by the processor 304, enable a therapy response score algorithm 320, a therapy parameter(s) algorithm 322, and/or mapping 324. Such content, if provided as in instruction, may, in some embodiments, be organized into one or more applications, modules, packages, layers, or engines.

The therapy response score algorithm 320 enables the processor 304 to process one or more inputs about a user (obtained from, for example, the sensor 326, the user device 328, the neuromodulation therapy system 100, 200, the user interface 310, the communication interface 308, the database 330, and/or the cloud 334) for the purpose of, for example, determining one or more therapy response scores for a user. The one or more inputs may be received during a stimulation therapy and/or when a stimulation therapy has ended. Similarly, the one or more inputs may be received at one time or may be tracked over a period of time (whether a period of time each day for a predetermined number of days, months, or years). The one or more inputs may comprise physiological inputs (e.g., heart rate, heart rate variability, respiration, eCAP signal, etc.), user inputs (e.g., user activity, user pain, etc.), and/or inputs obtained or determined from sensor output of the sensor 326 (e.g., time of day, user activity, user movement, user location, whether a user is asleep, weather, etc.). More specifically, calculated heart rate variability metrics may include (but are not limited to) time series metrics, such as standard deviation N-N (SDNN) intervals of normal heart beats and the root mean squared of successive differences (RMSSD) between normal heart beats, frequency domain metrics, such as high-frequency and low frequency heart rate variability, and time-frequency metrics. These heart rate variability metrics provide different insights into autonomic nervous system function. For example, RMSSD and high frequency heart rate variability are associated with parasympathetic activity.

The output can include an initial therapy response score or an increasing therapy response score or a decreasing therapy response score in relation to the initial therapy response score. The therapy response score may correlate to a patient health state score or a well-being score, which may be indicative of patient's health state or overall health. The patient's health state may indicate how much therapy a patient needs for that day (e.g. more therapy may be utilized if patient's health state is lower).

The therapy parameters algorithm 322 enables the processor 304 to process one or more inputs about the user (obtained from, for example, the sensor 326, the user device 328, the neuromodulation therapy system 100, 200, the user interface 310, the communication interface 308, the database 330, and/or the cloud 334) for the purpose of, for example, determining an adjustment to one or more parameters of a neuromodulation therapy (e.g., the neuromodulation therapy system 100, 200) for the user. In some embodiments, the one or more inputs may comprise the therapy response score obtained from the therapy response score algorithm 320. In other embodiments, the one or more inputs may include any combination of the therapy response score, physiological inputs, user inputs, and/or inputs obtained or determined from sensor output of the sensor 326. Adjusting the one or more parameters may include one or more of beginning or stopping a cycling of therapy, increasing or decreasing the cycling of therapy, increasing or decreasing the cycling of high-frequency stimulation, changing the ON/OFF cycling times while maintaining a cycling ratio, and/or adjustment of charge balancing strategy or recharge settings (e.g., how charge balance is achieved, such as making recharge adjustments between active recharge, passive recharge, a combination of active and passive recharge, frequency, duration, or any type of recharge). Additionally, adjusting one or more parameters may also include one or more of changing stimulation amplitude, frequency, pulse width, or electrode contact. It will be appreciated that in some embodiments the therapy parameters algorithm 322 may output the adjustments to the one or more parameters. In such embodiments, the one or more parameters may be adjusted manually by a user such as, for example, the patient, a medical provider, or clinician.

The mapping 324 enables the processor 304 to process one or more inputs as changed over a period of time (obtained from, for example, the sensor 326, the user device 328, the neuromodulation therapy system 100, 200, the user interface 310, the communication interface 308, the database 330, and/or the cloud 334) for the purpose of, for example, mapping the one or more inputs to one or more events, which may be, for example, diseases (e.g., cardiac diseases). The mapping may indicate that the user has experienced one or more events. For example, changes in a user's cardiac electrograms signals (as recorded by, for example, the neuromodulation therapy system 100, 200) may indicate a cardiac arrythmias and/or a type of arrythmias in the user. Such mapping may trigger a notification to the user to inform the user of the arrythmias and/or to seek a medical assessment.

The memory 306 may store other types of content or data (e.g., machine learning models, artificial neural networks, deep neural networks, etc.) that can be processed by the processor 304 to carry out the various method and features described herein. Thus, although various contents of memory 306 may be described as instructions, it should be appreciated that functionality described herein can be achieved through use of instructions, algorithms, and/or machine learning models. The data, algorithms, and/or instructions may cause the processor 304 to manipulate data stored in the memory 306 and/or received from or via the sensor 326, the user device 328, the neuromodulation therapy system 100, 200, the database 330, and/or the cloud 334.

The computing device 302 may also comprise a communication interface 308. The communication interface 308 may be used for receiving inputs or other information from an external source (such as the sensor 326, the user device 328, the neuromodulation therapy system 100, 200, the database 330, the cloud 334, and/or any other system or component not part of the system 300), and/or for transmitting instructions, inputs, or other information to an external system or device (e.g., another computing device 302, the sensor 326, the user device 328, the neuromodulation therapy system 100, 200, the database 330, the cloud 334, and/or any other system or component not part of the system 300). The communication interface 308 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 308 may be useful for enabling the device 302 to communicate with one or more other processors 304 or computing devices 302, whether to reduce the time needed to accomplish a computing-intensive task or for any other reason.

The computing device 302 may also comprise one or more user interfaces 310. The user interface 310 may be or comprise a keyboard, mouse, trackball, monitor, television, screen, touchscreen, and/or any other device for receiving information from a user and/or for providing information to a user. The user interface 310 may be used, for example, to receive a user selection or other user input regarding any step of any method described herein. Notwithstanding the foregoing, any required input for any step of any method described herein may be generated automatically by the system 300 (e.g., by the processor 304 or another component of the system 300) or received by the system 300 from a source external to the system 300. In some embodiments, the user interface 310 may be useful to allow a surgical provider or other user to modify instructions to be executed by the processor 304 according to one or more embodiments of the present disclosure, and/or to modify or adjust a setting of other information displayed on the user interface 310 or corresponding thereto.

Although the user interface 310 is shown as part of the computing device 302, in some embodiments, the computing device 302 may utilize a user interface 310 that is housed separately from one or more remaining components of the computing device 302. In some embodiments, the user interface 310 may be located proximate one or more other components of the computing device 302, while in other embodiments, the user interface 310 may be located remotely from one or more other components of the computer device 302.

Sensor(s) 326 may be configured to provide one or more sensor outputs. The sensors 326 may correspond to transducers that are configured to convert physical phenomena into an electrical signal that is capable of being processed by the processor 304 or any other processor (e.g., a processor of the neuromodulation system 100, 200, a processor of the user device 328, etc.). The sensor 326 may include one or more or any combination of components that are electrical, mechanical, electro-mechanical, magnetic, electromagnetic, or the like. The sensor 326 may include, but is not limited to, one or more of a camera, a navigational camera, a temperature sensor, a torque sensor, a force sensor, a linear encoder, a rotary encoder, a capacitor, gyroscopic sensors, strain gauges, impedance sensors, impact sensors, vibration detectors, and/or an accelerometer. In some embodiments, the sensor 326 may include a memory for storing sensor data. In still other examples, the sensor 326 may transmit sensor output(s) (e.g., sensor data) to one or more sources (e.g., the computing device 302, the user device 328, and/or the neuromodulation therapy system 100, 200).

The sensor 326 may be positioned adjacent to or integrated with another component of the system 300 such as, but not limited to, the user device 328 and/or the neuromodulation therapy system 100, 200. In some embodiments, the sensor 326 is positioned as a standalone component. The sensor 326 may include a plurality of sensors and each sensor may be positioned at the same location or a different location as any other sensor. It will be appreciated that in some embodiments the sensor(s) 326 can be positioned at or on any component of the system 300 or environment (e.g., on any portion of the user device 328 and/or the neuromodulation therapy system 100, 200).

The sensor 326 may send the sensor output or the sensor data to the computing device 302 continuously, at an interval, when prompted by user input, and/or when one or more conditions are sensed (e.g., a user is asleep, user movement, a change in temperature, a change in the environment, etc.). Further, in some embodiments, the sensor 326 may send data to the computing device 302 to display on the user interface 110 or otherwise notify the surgeon or operator of the change in the characteristic. In other embodiments, the sensor 326 may alert the user of the change in the characteristic by an alert such as, but not limited to, a sound or a light display.

The user device 328 may be configured to track and/or record one or more environmental conditions (e.g., time of day, temperature, elevation, battery charge, etc.), physiological responses (heart rate, heart rate variability, user temperature, etc.), user inputs (pain responses, when the user is performing an activity, when the user is asleep, a start and/or end of a user's menstrual cycle, etc.), and/or user conditions (e.g., user activity, user sleep cycle, etc.). The user device 328 may comprise, for example, an epilepsy device, a smart phone, a mobile phone, a wearable device, a smart wearable device, or the like. The user device 328 may communicate with any component of the system 300 and may send or receive data from the sensor 326, the computing device 302, the neuromodulation therapy system 100, 200, or any other component outside of the system 300. It will be appreciated that the user device 328 may comprise more than one user device 328 (e.g., a user may use a smart phone and a smart wearable device).

The database 330 may store information such as user data, results of a stimulation and/or neuromodulation procedure, stimulation and/or neuromodulation parameters, electrical signal parameters, electrode parameters, etc. The database 330 may be configured to provide any such information to the computing device 302, the neuromodulation therapy system 100, 200, or to any other device of the system 300 or external to the system 300, whether directly or via the cloud 334. In some embodiments, the database 330 may be or comprise part of a hospital image storage system, such as the PACS, the HIS, and/or another system for collecting, storing, managing, and/or transmitting electronic medical records.

The cloud 334 may be or represent the Internet or any other wide area network. The computing device 302 may be connected to the cloud 334 via the communication interface 308, using a wired connection, a wireless connection, or both. In some embodiments, the computing device 302 may communicate with the database 330 and/or an external device (e.g., a computing device) via the cloud 334.

It will be appreciated that the one or more inputs (whether physiological, environmental, etc.) may be received from any component of the system 300 or the neuromodulation therapy systems 100, 200 and the one or more inputs may be stored or processed by any component the system 300 or the neuromodulation therapy systems 100, 200. For example, the one or more inputs may be received from the neuromodulation therapy system 100, 200, the sensors 326, and/or the user device 328 by the computing device 302. The computing device 302 may store and/or process the one or more inputs (using, for example, the processor 304 and storing in the memory 306.). In other instances, the one or more inputs may be transmitted to and processed by the cloud 334 or the user device 328. In still other embodiments, the one or more inputs may be stored in the database 330. Further, the one or more inputs or an analysis of the one or more inputs may be outputted to the neuromodulation therapy systems 100, 200, or any other component (for example, the user device 328). In other embodiments, the one or more inputs or an analysis of the one or more inputs may be displayed to a user such as the patient, the clinician, or medical provider via, for example, the user device 328 or the user interface 310. For example, one or more suggested adjustments or therapy response scores may be displayed to the user. It will be appreciated that the one or more inputs may be received, processed, stored, and/or outputted by various components which may be parts of different components or may be integrated into one component.

The system 300 or similar systems may be used, for example, to carry out one or more aspects of any of the methods 500, 600, 700, and/or 800 described herein. The system 300 or similar systems may also be used for other purposes.

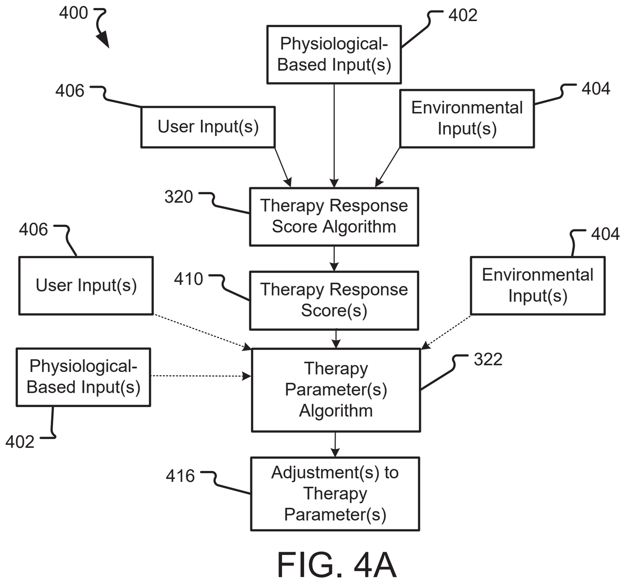

Turning to Fig. 4A, an example of a model architecture 400 that supports methods and systems (e.g., Artificial Intelligence (AI)-based methods and/or system) for generating therapy response scores and adjustments to therapy parameter(s) is shown.

Physiological-based inputs of a user 402, user inputs 406, and/or environmental inputs of an environment surrounding the user 404 may be used by a processor such as the processor 304 as input for a therapy response score algorithm 320. It will be appreciated that the input may not include the user inputs 406 and/or the environmental inputs 404. The therapy response score algorithm 320 may output one or more therapy response scores 410. In some embodiments, the physiological-based inputs 402 may be received from the neuromodulation therapy system 100, 200, the user device 328, the sensor 326, the computing device 302, the database 330, the cloud 334, and/or any component of a system such as the system 300 or the neuromodulation therapy system 100, 200. The physiological-based inputs 402 may comprise, for example, blood pressure, oxygen saturation (SpO2), impedance measurements, vital signs, cardiac electrograms and derived metrics (e.g., heart rate, heart rate variability, and/or respiration rate), activity information (e.g., accelerometry-derived information such as step count, steps/minute, a position of the user, a time stamp and/or period of time regarding a change in posture of the user), eCAP signals, evoked compound muscle action potential (ECMAP) activity, a pain score based on, for example, heart rate, heart rate variability, respiration rate, and/or a position and/or activity of the user, a user's menstrual cycle (obtained from, for example, cardiac electrogram sand derived metrics and/or user input), and/or a quality of sleep score (e.g., comprising one or more of: a total sleep time, a sleep onset latency, a number of positional changes of the user, time spent laying down by the user, time in a preferred sleep position by the user, number of interruptions of sleep of the user, time spent in a specific sleep stage, and/or depth of sleep of the user as defined by, for example, HR, respiration, etc.). The environmental inputs 404 may be received from the sensor 326, the user device 328, the computing device 302, the database 330, the cloud 334, and/or or any component of a system such as the system 300 or the neuromodulation therapy system 100, 200. The environmental inputs 404 may comprise, for example, a temperature, a time of day (relative to, for example, user sleep and/or user activity), an elevation, a humidity, the date, whether a battery of the device 102, 204 of the neuromodulation therapy system 100, 200 or any other battery needs recharging, and/or weather. The user inputs 406 may be received from the user device 328, the computing device 302, the database 330, the cloud 334, and/or or any component of a system such as the system 300 or the neuromodulation therapy system 100, 200. The user inputs 406 may comprise, for example, user inputs to a questionnaire, user input regarding an activity, user pain, user satisfaction with the neuromodulation therapy, and/or user input regarding a time of sleep.

It will be appreciated that the physiological-based inputs 402, the user inputs 406, and/or the environmental inputs 404 may be received from multiple devices and that an individual input can be received from multiple devices (e.g., heart rate is received from a wearable device worn by the user and a neuromodulation therapy system implanted in the user). Where the input is received from multiple sources, an input from a preferred device may be prioritized over a secondary device or the input from multiple devices may be averaged. Further, the physiological-based inputs 402, the user inputs 406, and/or the environmental inputs 404 may be received at different stages of the neuromodulation therapy such as, for example, during pre-trial of a test neuromodulation therapy system (e.g., input(s) may be received from a wearable device or other user device), during a trial/testing of a test neuromodulation therapy system, post-trial/testing of the test neuromodulation therapy system, and/or during or after implantation of a permanent neuromodulation therapy system. Additionally, the physiological-based inputs 402, the user inputs 406, and/or the environmental inputs 404 may be received over different periods of time. In some embodiments, the physiological-based inputs 402, the user inputs 406, and/or the environmental inputs 404 may be tracked during stimulation by the neuromodulation therapy system 100, 200 and/or without stimulation to help determine the therapy response score 410 and/or an adjustment to at least one therapy parameter 416. Additionally or alternatively, the physiological-based inputs 402, the user inputs 406, and/or the environmental inputs 404 may be tracked during different activities of the user. For example, the inputs may be tracked when a user is active (e.g., running or walking) and may then be tracked when the user is sleeping.

The therapy response score algorithm 320 may use the physiological-based inputs 402, the user inputs 406, and/or the environmental inputs 404 to output one or more therapy response scores 410. The therapy response score algorithm 320 may weight one or more of the inputs to determine the score. For example, the therapy response score algorithm 320 may weigh the user's heart rate higher during an activity than when the user is sedentary. The one or more therapy response scores 410 may correlate to whether a neuromodulation therapy is sufficient for the user, or whether one or more parameters of the neuromodulation therapy can be adjusted (whether manually or automatically) to improve the neuromodulation therapy. In other words, the therapy response score 410 correlates to whether a neuromodulation therapy is sufficient for a user and enables adjusting and customizing the neuromodulation therapy for an individual user.

The therapy response score algorithm 320 may be trained using historical physiological-based inputs, historical user inputs, and/or historical environmental inputs. In other embodiments, the therapy response score algorithm 320 may be trained using the physiological-based inputs 402, the user inputs 406, and/or the environmental inputs 404. In such embodiments, the therapy response score algorithm 320 may be trained prior to inputting the physiological-based inputs 402, the user inputs 406, and/or the environmental inputs 404 into the therapy response score algorithm 320 or may be trained in parallel with inputting the physiological-based inputs 402, the user inputs 406, and/or the environmental inputs 404 into the therapy response score algorithm 320. As described, the therapy response score algorithm 320 may output the one or more therapy response scores 410 that may be used as input for a therapy parameters algorithm 322.

In some embodiments, the physiological-based inputs 402, the user inputs 406, and/or the environmental inputs 404 may be used by a processor such as the processor 304 as input for the therapy parameters algorithm 322. In other embodiments, the therapy response scores 410 may be used by a processor such as the processor 304 as input for the therapy parameters algorithm 322. In still other embodiments, any combination of the therapy response scores 410, the physiological-based inputs 402, the user inputs 406, and/or the environmental inputs 404 may be used by a processor such as the processor 304 as input for the therapy parameters algorithm 322. The therapy parameters algorithm 322 may output an adjustment to at least one therapy parameter 416. The adjustment can be implemented automatically (by, for example, the computing device 302 or a processor such as the processor 304) or manually (by, for example, a user such as a patient, a clinician, or medical provider). The therapy response score 410 may be received from the therapy response score algorithm 320, though it will be appreciated that the therapy response score 410 can be received from any component such as, for example, the user device 328, the computing device 302, the database 330, and/or the cloud 334.

The therapy parameters algorithm 322 may use the therapy response score 410, the physiological-based inputs 402, the user inputs 406, and/or the environmental inputs 404 to output the adjustment to the at least one therapy parameter 416. The therapy parameters algorithm 322 may also weight one or more of the inputs to determine the score. The therapy parameters algorithm 322 may additionally determine whether the at least one therapy parameter should be adjusted. For example, the therapy response score may indicate that current parameters for a user are sufficient and/or satisfactory and thus the current parameters do not need to be adjusted. In another example, it may be desirable to obtain information such as the physiological inputs of a user, and thus the therapy parameters algorithm 322 may not be used. Adjusting the at least one therapy parameter may comprise, for example, changing stimulation amplitude, frequency, pulse width, or electrode contact, adjusting one or more of beginning or stopping a cycling of therapy, increasing or decreasing the cycling of therapy, increasing or decreasing the cycling of high-frequency stimulation, changing the ON/OFF cycling times while maintaining a cycling ratio, and/or adjustment of charge balancing strategy or recharge settings (e.g., how charge balance is achieved, such as making recharge adjustments between active recharge, passive recharge, a combination of active and passive recharge, frequency, duration, or any type of recharge). The adjustment to the at least one therapy parameter 416 can be used to customize the neuromodulation therapy for a user.

The therapy parameters algorithm 322 may be trained using historical therapy response scores, historical physiological-based inputs, historical user inputs, and/or historical environmental inputs. In other embodiments, the therapy parameters algorithm 322 may be trained using the therapy response score 410, the physiological-based inputs 402, the user inputs 406, and/or the environmental inputs 404. In such embodiments, the therapy parameters algorithm 322 may be trained prior to inputting the therapy response score 410, the physiological-based inputs 402, the user inputs 406, and/or the environmental inputs 404 into the therapy parameters algorithm 322 or may be trained in parallel with inputting the therapy response score 410, the physiological-based inputs 402, the user inputs 406, and/or the environmental inputs 404 into the therapy parameters algorithm 322.

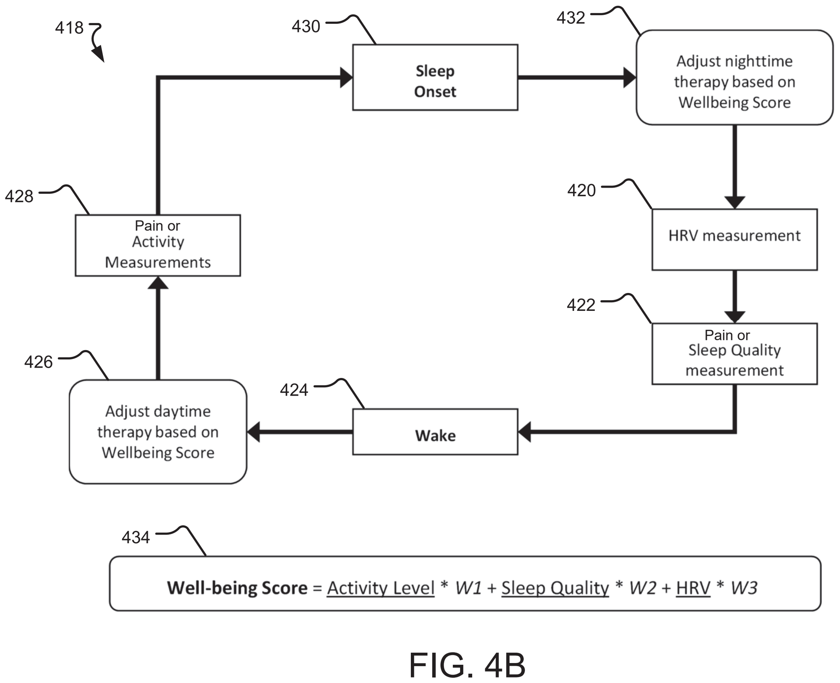

Turning to Fig. 4B, an example feedback loop 418 based on a user's overall well-being or health state according to at least one embodiment of the present disclosure is provided. The feedback loop 418 may be repeated continuously. In other embodiments, parts of the feedback loop 418 may be repeated.

The feedback loop 418 may include receiving or obtaining cardiac measurements such as HRV measurements 420 (e.g., heart rate, heart rate variability, and/or respiration rate) and/or sleep quality measurements 422 (e.g., a number of positional changes of the user, time spent laying down by the user, time in a preferred sleep position by the user, number of interruptions of sleep of the user, and/or depth of sleep of the user as defined by, for example, HR, respiration, etc.). The HRV measurements 420 and/or the sleep quality measurements 422 may be measured or obtained for a period of time correlating to when the user is asleep (e.g. a period of sleep time). Alternatively or additionally, a pain score based on, for example, heart rate, heart rate variability, respiration rate, and/or a position and/or activity of the user may also be obtained. The position and/or the activity of the user may be obtained from, for example, the sensor such as an accelerometer, a position sensor, a wearable device, etc. The HRV measurements 420 and/or the sleep quality measurements 422 may be measured continuously throughout the period of sleep time, intermittently throughout the period of sleep time, or at different intervals throughout the period of sleep time. The HRV measurements 420 and/or the sleep quality measurements 422 may also be used as input to, for example, the therapy response score algorithm 320 to output, for example, a therapy response score 410 such as a well-being score. The well-being score may alternately or additionally be obtained by an equation 434 wherein the well-being score equals the activity measurements 428 times a first weight plus the sleep quality measurements 422 times a second weight plus the HRV measurements 420 times a third weight. When the user wakes at a wake stage 424, the HRV measurements 420 and/or the sleep quality measurements 422 may be used to determine or provide adjustments to daytime therapy based on the well-being score 424 (which may use, for example, the therapy parameters algorithm 322 to determine one or more adjustments to the therapy parameters 416).

While the user is awake, activity measurements 428 (e.g., accelerometry-derived information such as step count, steps/minute, a position of the user, a time stamp and/or period of time regarding a change in posture of the user) may be obtained or received throughout another period of time correlating to when the user is awake (e.g., a period of wake time). Alternatively or additionally, the pain score based on, for example, heart rate, heart rate variability, respiration rate, and/or a position and/or activity of the user may also be obtained. The activity measurements 428 may be measured continuously throughout the period of wake time, intermittently throughout the period of wake time, or at different intervals throughout the period of wake time. The activity measurements 428 may also be used as input to, for example, the therapy response score algorithm 320 to output, for example, a therapy response score 410 such as a well-being score As previously described, the well-being score may alternately or additionally be obtained by the equation 434. When the user sleeps at a sleep onset stage 430, the activity measurements 428 may be used to determine or provide adjustments to the nighttime therapy based on the well-being score 432 as determined using the activity measurements 428 (which may use, for example, the therapy parameters algorithm 322 to determine one or more adjustments to the therapy parameters 416). The feedback loop 418 may then proceed to obtaining or receiving the HRV measurements and/or the sleep quality measurements 422.

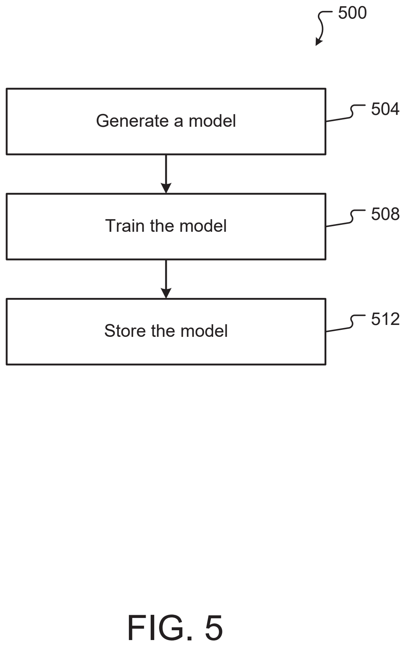

Fig. 5 depicts a method 500 that may be used, for example, for generating a model is provided.

The method 500 comprises generating a model (step 504). The model may be the therapy response score algorithm 320 and/or therapy parameters algorithm 322. A processor such as the processor 304 may generate the model. The model may be generated to facilitate and enable, for example, determining a therapy response score and an adjustment to one or more parameters of a neuromodulation therapy for the purpose of customizing the neuromodulation therapy for an individual user.

The method 500 also comprises training the model (step 508). In embodiments where the model is trained prior to implementation of the neuromodulation therapy for the user, the model may be trained using historical data from a number of users. In some embodiments, the historical data may be obtained from users that have similar user data to a user in which a neuromodulation therapy is to be implemented thereon. In other embodiments, the historical data may be obtained from any user.

In other embodiments, the model may be trained in parallel with use of another model. Training in parallel may, in some embodiments, comprise training a model using input received during, for example, or prior to implantation of the neuromodulation therapy, while also using a separate model to receive and act upon the same input. Such input may be specific to a user upon which the neuromodulation therapy is to be implemented thereon. In some instances, when the model being trained exceeds the model in use (whether in efficiency, accuracy, or otherwise), the model being trained may replace the model in use. Such parallel training may be useful, for example, in situations, where a model is continuously in use (for example, when the input(s) are continuously received throughout use of a neuromodulation therapy system 100, 200 implementing the neuromodulation therapy) and a corresponding model may be trained in parallel for further improvements.

In some embodiments, it will be appreciated that the model trained using historical data may be initially used as a primary model at a start of the neuromodulation therapy. A training model may also be trained in parallel with the primary model using user-specific input until the training model is sufficiently trained. The primary model may then be replaced by the training model.

The method 500 also comprises storing the model (step 512). The model may be stored in memory such as the memory 306 and/or a database such as the database 330 for later use. In some embodiments, the model is stored in the memory when the model is sufficiently trained. The model may be sufficiently trained when the model produces an output that meets a predetermined threshold, which may be determined by, for example, a user, or may be automatically determined by a processor such as the processor 304.

The present disclosure encompasses embodiments of the method 500 that comprise more or fewer steps than those described above, and/or one or more steps that are different than the steps described above.

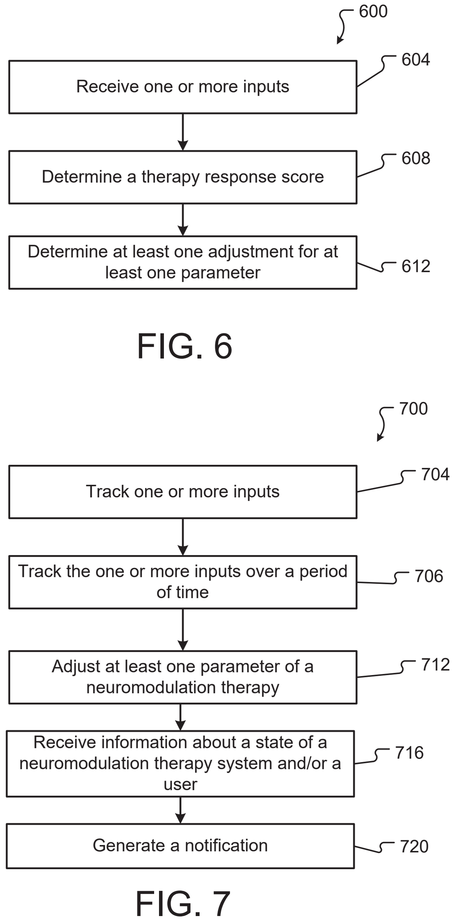

Fig. 6 depicts a method 600 that may be used, for example, for adjusting one or more parameters of a neuromodulation therapy.

The method 600 (and/or one or more steps thereof) may be carried out or otherwise performed, for example, by at least one processor. The at least one processor may be the same as or similar to the processor(s) 304 of the computing device 302 described above. The at least one processor may be part of a neuromodulation therapy system such as the neuromodulation therapy system 100, 200 (which may be open-loop or closed-loop). A processor other than any processor described herein may also be used to execute the method 600. The at least one processor may perform the method 600 by executing elements stored in a memory such as the memory 306. The elements stored in the memory and executed by the processor may cause the processor to execute one or more steps of a function as shown in method 600. One or more portions of a method 600 may be performed by the processor executing any of the contents of memory, such as a therapy response score algorithm 320, a therapy parameters algorithm 322, and/or a mapping 324.

The method 600 comprises receiving one or more inputs (step 604). The one or more inputs may comprise physiological-based inputs of a user such as the physiological-based inputs 402, user inputs such as the user inputs 406, and/or environmental inputs of an environment surrounding the user such as the environmental inputs 404.

As previously described, the physiological-based inputs may be received from a neuromodulation therapy system such as the neuromodulation therapy system 100, 200 (as recorded by, for example, recording electrodes such as the recording electrodes 210), a user device such as the user device 328, a sensor such as the sensor 326, a computing device such as the computing device 302, a database such as the database 330, a cloud such as the cloud 334, and/or any component of a system such as the system 300 or the neuromodulation therapy system. The physiological-based inputs may comprise, for example, vital signs, blood pressure, oxygen saturation (SpO2), impedance measurements, cardiac electrograms and derived metrics (e.g., heart rate, heart rate variability, and/or respiration rate), activity information (e.g., accelerometry-derived information such as step count, steps/minute, a position of the user, a time stamp and/or period of time regarding a change in posture of the user), eCAP signals (whether recorded as active or passive), evoked compound muscle action potential (ECMAP) activity, and/or a quality of sleep score (e.g., a number of positional changes of the user, time spent laying down by the user, time in a preferred sleep position by the user, number of interruptions of sleep of the user, and/or depth of sleep of the user as defined by, for example, HR, respiration, etc.). The environmental inputs may be received from the sensor, the user device, the computing device, the database, the cloud, and/or or any component of the system or the neuromodulation therapy system. The environmental inputs may comprise, for example, a temperature, a time of day (relative to, for example, user sleep and/or user activity), an elevation, a humidity, the date, whether a battery of the device 102, 204 of the neuromodulation therapy system 100, 200 or any other battery needs recharging, and/or weather. The user inputs may be received from the user device, the computing device, the database, the cloud, and/or or any component of the system or the neuromodulation therapy system. The user inputs may comprise, for example, user inputs to a questionnaire, user input regarding an activity, user pain, user satisfaction with the neuromodulation therapy, and/or user input regarding a time of sleep. The inputs may also include a pain score based on, for example, heart rate, heart rate variability, respiration rate, and/or a position and/or activity of the user. The position and/or the activity of the user may be obtained from, for example, the sensor such as an accelerometer, a position sensor, a wearable device, etc. The inputs may further include a user's menstrual cycle (obtained from, for example, cardiac electrogram sand derived metrics and/or user input), which may be repeatedly tracked on a monthly cycle.

It will be appreciated that the one or more inputs may be received from multiple devices and that an individual input can be received from multiple devices (e.g., heart rate is received from a wearable device worn by the user and a neuromodulation therapy system implanted in the user). Where the input is received from multiple sources, an input from a preferred device may be prioritized over a secondary device or the input from multiple devices may be averaged. Further, the one or more inputs may be received at different stages of the neuromodulation therapy such as, for example, during pre-trial of a test neuromodulation therapy system (e.g., input(s) may be received from a wearable device or other user device), during a trial/testing of a test neuromodulation therapy system, post-trial/testing of the test neuromodulation therapy system, and/or during or after implantation of a permanent neuromodulation therapy system. Additionally, the one or more inputs may be received over different periods of time. In some embodiments, the one or more inputs may be tracked during stimulation by the neuromodulation therapy system and/or without stimulation. Additionally or alternatively, the one or more inputs may be tracked during different activities of the user. For example, the inputs may be tracked when a user is active (e.g., running or walking) and may then be tracked when the user is sleeping.

The method 600 also comprises determining a therapy response score (step 608). The therapy response score may be determined by a processor such as the processor 304 executing a therapy response score algorithm such as the therapy response score algorithm 320. The one or more inputs (received in, for example, the step 604) may be received as input to the therapy response score algorithm and the therapy response score algorithm may output one or more therapy response scores. The therapy response scores may correlate to whether a neuromodulation therapy is sufficient for the user, or whether one or more parameters of the neuromodulation therapy can be adjusted to improve the neuromodulation therapy. In other words, the therapy response score correlates to whether a neuromodulation therapy is sufficient for a user and enables adjusting and customizing the neuromodulation therapy for an individual user. Further, the therapy response score is customized or tailored to individual users. For example, a user may have a low therapy response score comprised of a low heart rate variability (e.g., parasympathetic metrics), high resting heart rate, and low activity for a person in a chronic pain state experiencing high pain. The same user, when responding to the therapeutic levels of a stimulation therapy may have a high therapy response score comprised of an increased heart rate variability (e.g., parasympathetic metrics), a lower resting heart rate, and moderate activity levels compared to a baseline for the user. Such therapy response core may also be based on quality of sleep of the user, which may be based on lower respiration rates, lower heart rate, and laying positions of the user at evening hours.

In some embodiments, the therapy response score may comprise an individualized pain score based on input comprising cardiac-based metrics such as heart rate, heart rate variability, and cardiac-derived respiration. In such embodiments, the cardiac-driven metrics may be measured in specific positions of the user or movements of the user as detected by, for example, an accelerometer and may be used to determine the individualized pain score. For example, the heart rate and the heart rate variability may be measured when the user is laying down and when the accelerometer detects that the user has sat up, changes in the heart rate and the heart rate variability may be recorded and used to determine the individualized pain score. Alternatively, or additionally, the difference in the heart rate and the heart rate variability may be recorded when the user is still as compared to when the user is moving and/or when the user has stopped moving.

The method 600 also comprises determining at least one adjustment for at least one parameter of a therapeutic neuromodulation (step 612). The at least one adjustment may be based on the therapy response score determined in the step 608. In some embodiments, the step 612 may occur when the therapy response score is less than a threshold therapy response score. In other embodiments, the step 612 may occur when the therapy response score is greater than the threshold therapy response score. Similarly, it will be appreciated that in some embodiments, the method 600 may not include the step 612 such as, for example, when the therapy response score is sufficient for a user. For example, if it is desired to increase the therapy response score, changes in frequency, amplitude, the electrode being stimulated, and/or cycling may be adjusted. In another example, if it is desired to decrease the therapy response score, the cycling may be increased, the amplitude may be decreased, and/or the frequency of stimulation may be decreased. In still other embodiments, the method 600 may not include the step 612.

The adjustment may be determined by a processor executing a therapy parameters algorithm such as the therapy parameter algorithm 322. It will be appreciated that the adjustment may be implemented manually or automatically. In some embodiments a computing device such as the computing device 302 or a processor such as the processor 304 (or any processor) may automatically adjust the at least one parameter using the adjustment. In other embodiments, the at least one parameter may be adjusted by a user such as, for example, a patient, a clinician, or a medical provider using the adjustment. In such embodiments, the at least one adjustment may be displayed or otherwise outputted to the user by, for example, a user device such as the user device 328 and/or a user interface such as the user interface 310. The one or more inputs (received in, for example, the step 604) and/or the therapy response scores (received in, for example, the step 612) may be received as input to the therapy parameter algorithm 322 and the therapy parameter algorithm 322 may output adjustment(s) for one or more parameters. The adjustments for the at least one parameter may include, for example, changing stimulation amplitude, frequency, pulse width, or electrode contact, adjusting one or more of beginning or stopping a cycling of therapy, increasing or decreasing the cycling of therapy, increasing or decreasing the cycling of high-frequency stimulation, and/or changing the ON/OFF cycling times while maintaining a cycling ratio. The limits of cycling changes may be limited by user input (received from, for example, a programmer, a medical provider, etc.) to have a minimum and/or maximum ratio. The cycling may also be adjusted to affect parts of the stimulation, for example, only cycling targeting high frequency components and/or low frequency components in stimulation. The adjustments may also include adjustment of charge balancing strategy or recharge settings (e.g., how charge balance is achieved, such as making recharge adjustments between active recharge, passive recharge, a combination of active and passive recharge, frequency, duration, or any type of recharge), a change to when a battery of a device such as the device 102, 204 of the neuromodulation therapy system is recharged, how often the battery is recharged, and/or a duration of the battery recharge to optimize the parameters of the therapy, and thus optimize the therapy as experienced by the user.

It will be appreciated that any one of the steps of the method 600 may be repeated. For example, the steps 604, 608, and 612 may be repeated until the therapy response score for a user increases or decreases to a desired score. Thus, the method 600 can be used or enable customizing and adjusting the neuromodulation therapy for individual users.

The present disclosure encompasses embodiments of the method 600 that comprise more or fewer steps than those described above, and/or one or more steps that are different than the steps described above.

Fig. 7 depicts a method 700 that may be used, for example, for adjusting one or more parameters of a neuromodulation therapy based on tracking one or more physiological-based inputs.

The method 700 (and/or one or more steps thereof) may be carried out or otherwise performed, for example, by at least one processor. The at least one processor may be the same as or similar to the processor(s) 304 of the computing device 302 described above. The at least one processor may be part of a neuromodulation therapy system such as the neuromodulation therapy system 100, 200 (which may be open-loop or closed-loop). A processor other than any processor described herein may also be used to execute the method 700. The at least one processor may perform the method 700 by executing elements stored in a memory such as the memory 306. The elements stored in the memory and executed by the processor may cause the processor to execute one or more steps of a function as shown in method 700. One or more portions of a method 700 may be performed by the processor executing any of the contents of memory, such as a therapy response score algorithm 320, a therapy parameters algorithm 322, and/or a mapping 324.

The method 700 comprises tracking one or more inputs (step 704). The one or more inputs may be the same as or similar to the one or more inputs described in the step 604 of the method 600 above. For example, the one or more inputs may comprise physiological-based inputs of a user such as the physiological-based inputs 402, user inputs such as the user inputs 406, and/or environmental inputs of an environment surrounding the user such as the environmental inputs 404.

The one or more inputs may be tracked at a first time to record a base or initial set of one or more inputs unique to the user. In some embodiments, the one or more inputs may be tracked during stimulation by the neuromodulation therapy system and/or without stimulation. The one or more inputs may be tracked by any component of a neuromodulation therapy system such as the neuromodulation therapy system 100, 200 and/or any component of a system such as the system 300. For example, the one or more inputs may be tracked by recording electrodes such as the recording electrodes 210, a user device such as the user device 328, sensors such as the sensors 326, and/or a computing device such as the computing device 302. The tracked one or more inputs may be stored in, for example, a memory such as the memory 306, a database such as the database 330, and/or a cloud such as the cloud 334.

The method 700 also comprises tracking the one or more inputs over a period of time (step 708). The step 708 may be the same as or similar to the step 704 and the one or more inputs may be tracked after the first time and over the period of time to yield one or more changed inputs. The tracking can be repeated over two or more days for the period of time each day. The period of time can vary from hours (such that, for example, optimal stimulation cycling is found for nighttime vs. day time) to days, weeks, or months (to, for example, help inform long-term needs to changes in the neuromodulation therapy for the user).

In some embodiments, the period of time can comprise a first period of time, a second period of time, and/or any number of period of times. For example, a first period of time can correspond to when a user is awake and the second period of time can correspond to when the user is asleep. Alternatively, or additionally, the first period of time can correspond to when the user is active and the second period of time can correspond to when the user is inactive. Tracking at different periods of time can be used to track, for example, inputs at different periods of rest or inputs at different periods of activities (whether in the same day or over the course of multiple days). It will be appreciated that the tracking can also be adjusted, changed, or determined based on the one or more inputs and/or input about the user's state (whether determined from user input, sensor output, etc.). For example, the one or more inputs can indicate that a user is active (e.g., accelerometer data may indicate that the user is running) and can begin a period of time and can end the period of time when the one or more inputs indicates that the user has completed the activity.

The method 700 also comprises adjusting at least one parameter of the neuromodulation therapy (step 712). The step 712 may be the same as or similar to the step 612 of the method 600 above with respect to adjusting the at least one parameter. In some embodiments, the at least one parameter may be adjusted based on a difference between the one or more inputs and the one or more changed inputs. It will be appreciated that in some embodiments, the method 700 may not include the step 712, whether because the neuromodulation therapy system is simply used to output information such as physiological input(s) about the user or it is determined that the one or more inputs does not need to be adjusted based on the tracking in the step 706. It will also be appreciated that adjusting the at least one parameter be implemented manually or automatically. In some embodiments a computing device such as the computing device 302 or a processor such as the processor 304 (or any processor) may automatically adjust the at least one parameter using the adjustment. In other embodiments, the at least one parameter may be adjusted by a user such as, for example, a patient, a clinician, or a medical provider using the adjustment.

The method 700 also comprises receiving information about a state of the neuromodulation therapy system and/or a state of the user (step 716). The information about the state of the neuromodulation system may be received from the neuromodulation therapy system or as user input from the user. The information about the state of the neuromodulation system may include a battery lifespan of the neuromodulation therapy system, system performance metrics, and/or any detected failures or malfunctions of any component of the neuromodulation therapy system.

The state of the user may be received as user input from the user device or the computing device. In other embodiments, the state of the user may be based one or more events of the user determined from the changed one or more inputs (tracked in, for example, the step 708). More specifically, the one or more events may be determined by the processor executing a mapping such as the mapping 324 to map one or more changed inputs tracked over the period of time to one or more events. The one or more events may comprise, for example, respiration suppression event, a seizure, abnormal heart arrythmias, sleep apnea, an imbalance in the user (which can predict a fall using information from, for example, an accelerometer), increased temperature indicating a potential infection, or the like. In one example, the heart arrythmias may be mapped based on cardiac electrograms (which may indicate, for example, abnormal or non-regular rhythm of the heart rate) and derived metrics recorded from the neuromodulation therapy system and positional data of the user (e.g., whether the user is sitting, standing, etc.) determined from an accelerometer. The positional data may be useful in determining false errors, particularly during user movement, as the cardiac electrograms and derived metrics may change shape or quality depending on a user position and/or user movement.

The method 700 also comprises generating a notification (step 720). The notification can be generated when the state of the neuromodulation therapy system and/or the state of the user meets or exceeds a state threshold. For example, the notification may be generated when a battery level of the neuromodulation therapy system is low. In another example, the notification may be generated when the user has experienced sleep apnea or any respiration suppression event. In still another example, the notification can be generated when a user has a seizure. In such examples, the user may have a user device such as the user device 328 which may comprise, for example, an epilepsy device. The notification may be transmitted to the user device and/or may be generated by the user device. Further, the notification may be transmitted to any other device such as the neuromodulation therapy system, another neuromodulation therapy system, and/or another user device. It will be appreciated that the method 700 may not include the step 720 in cases where, for example, the state of the neuromodulation therapy system and the state of the user are stable.

The notification may be a visual notification, an audible notification, or any type of notification communicated to a user. The notification may be communicated to the user via a user interface such as the user interface 310. Alternatively, or additionally, the notification may be communicated to the user via the user device and may be, for example, a vibration, an audible sound, text (e.g., description of the event, suggestions to the user to seek help, sit down, or find support, etc.). The notification may also be automatically sent to an emergency provider in cases of an emergency (e.g., a fall, a cardiac event, or the like). In some instances, the neuromodulation therapy system may provide the notification via, for example, vibrations and/or stimulations. The vibrations and/or the stimulation can be delivered in specific patterns and the stimulation can be delivered in specific frequencies (e.g., low frequency stimulation) to prompt the user to check their user device for a notification. In some embodiments, the notification may be automatically generated by the processor. In other embodiments, the notification may be automatically generated by any component of a system such as the system 300.

The present disclosure encompasses embodiments of the method 700 that comprise more or fewer steps than those described above, and/or one or more steps that are different than the steps described above.

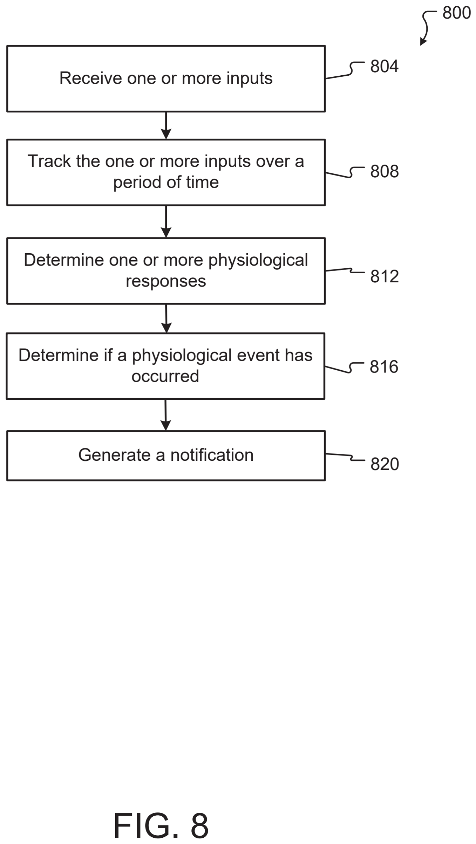

Fig. 8 depicts a method 800 that may be used, for example, for determining one or more physiological responses.

The method 800 (and/or one or more steps thereof) may be carried out or otherwise performed, for example, by at least one processor. The at least one processor may be the same as or similar to the processor(s) 304 of the computing device 302 described above. The at least one processor may be part of a neuromodulation therapy system such as the neuromodulation therapy system 100. A processor other than any processor described herein may also be used to execute the method 800, 200 (which may be open-loop or closed-loop). The at least one processor may perform the method 800 by executing elements stored in a memory such as the memory 306. The elements stored in the memory and executed by the processor may cause the processor to execute one or more steps of a function as shown in method 800. One or more portions of a method 800 may be performed by the processor executing any of the contents of memory, such as a therapy response score algorithm 320, a therapy parameters algorithm 322, and/or a mapping 324.

The method 800 comprises receiving one or more inputs (step 804). The step 804 may be the same as or similar to the step 604 of the method 600 described above. As previously described in the steps 604 and 704, the one or more inputs may be recorded by, for example, recording electrodes such as the recording electrode 210 of a neuromodulation therapy system such as the neuromodulation therapy system 100, 200. The recording electrodes may be on the same lead as stimulating electrodes such as the stimulating electrodes 206. In at least one example, a lead which may be the same as or similar to the lead 104, 208A, 208B may be implanted in the spinal cord and designed such that the recording electrodes are proximal to the stimulating electrodes such that the recording captures responses as it exits the epidural space and may target the fascia. In another example, the recording contacts are placed on another lead that is not implanted in the spinal cord. The one or more inputs may include cardiac signals and/or muscle activity signals that may or may not be elicited in response to electrical stimulation by the neuromodulation therapy system.

The method 800 also comprises tracking the one or more inputs over a period of time (step 808). The step 808 may be the same as or similar to the step 708 of the method 700 described above.

The method 800 also comprises determining one or more physiological responses (step 812). The one or more physiological responses may be determined based on a difference between the one or more inputs received or measured at a first time (as received in, for example, the step 804) and the one or more changed inputs as tracked over the period of time (as tracked in, for example, the step 808). The one or more physiological responses may comprise growth curves generated from tracked ECMAP activity, which may be used to determine a reduction or increase in stimulation and/or cycling of therapy, as described in the methods 600 and 700. The one or more physiological responses may also include changes in heart rate and/or heart rate variability as recorded during a resting phase of a user can also be used to adjust the reduction or increase in stimulation and/or cycling of therapy. Similarly, the one or more physiological responses may be used to determine if the lead has migrated, as discussed below.

The method 800 also comprises determining if a physiological event has occurred (step 816). Determining if a physiological event has occurred may be based on the one or more physiological responses. The physiological event may comprise, for example, whether the lead has migrated. In such examples, recording of ECMAP signals or changes in the ECMAP signals and/or the cardiac electrogram waveforms and/or changes in ECAP signals (whether active or passive) may indicate that the lead has migrated. Similarly, changes in one or more features of the cardiac electrogram waveform may indicate lead migration as the cardiac electrogram signal is known to change during a lead pull. Conversely, changes in noise characteristics (e.g., signal-to-noise ratio, the noise amplitude, or the spectral properties of the noise) may also be used to indicate lead migration. In still other embodiments, migration of the lead may be determined based on changes in amplitudes of the signals, (which may include sudden detection and/or loss of such signals), frequency content of these signals, or changes in the waveform shapes (which may be determined through, for example, template identification, principal component analysis, spectral analysis, wavelet analysis, or the like). In such instances where the lead has been determined to migrate based on the one or more physiological responses, a medical provider may confirm such migration using imaging such as fluoroscopic imaging.

It will be appreciated input received from other sources such as, for example, a sensor such as the sensor 326 may be used to determine if the lead has migrated. For example, an accelerometer may be used to determine changes in one or more inputs due to user activity and/or user positions rather than lead migration.

The method 800 also comprises generating a notification (step 824). The step 824 may be the same as or similar to the step 720 of the method 700 described above. The notification may be generated when the lead has been determined to have migrated from an initial position within the user.

The present disclosure encompasses embodiments of the method 800 that comprise more or fewer steps than those described above, and/or one or more steps that are different than the steps described above.

As noted above, the present disclosure encompasses methods with fewer than all of the steps identified in Figs. 5, 6, 7, and 8 (and the corresponding description of the methods 500, 600, 700, and 800), as well as methods that include additional steps beyond those identified in Figs. 5, 6, 7, and 8 (and the corresponding description of the methods 500, 600, 700, and 800). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Further disclosed herein is the subject-matter of the following clauses:
1. A system for generating a neuromodulation therapy of a user, the system comprising:
   a processor; and
   a memory storing data for processing by the processor, the data, when processed, causes the processor to:
      receive one or more physiological-based inputs of the user,
      determine a therapy response score based on the one or more physiological-based inputs, and
      generate at least one adjustment for one or more parameters of the neuromodulation therapy based on the therapy response score.
2. The system of clause 1, wherein the memory stores further data for processing by the processor that, when processed, causes the processor to:
   apply the at least one adjustment to the one or more parameters, wherein the one or more parameters are adjusted automatically or manually.
3. The system of clause 1 or of any of the preceding clauses, wherein the memory stores further data for processing by the processor that, when processed, causes the processor to:
   receive information about a state of a therapy system configured to deliver the neuromodulation therapy; and
   generate a notification based on at least one of the state of the therapy system, the therapy response score, or the one or more physiological-based inputs.
4. The system of clause 1 or of any of the preceding clauses, wherein the one or more parameters comprises at least one of increasing or decreasing a stimulation amplitude of a signal, increasing or decreasing a pulse width of a signal, increasing or decreasing a frequency of a signal, increasing or decreasing a number of electrode contacts, changing the electrode contacts that provide stimulation, starting or stopping the therapy, increasing or decreasing a cycling of the therapy, increasing or decreasing the cycling of high frequency components of the therapy independently of low frequency components of the therapy, adjustment of charge balancing strategy, or changing an on/off cycling time while maintaining a cycling ratio.
5. The system of clause 1 or of any of the preceding clauses, wherein the memory stores further data for processing by the processor that, when processed, causes the processor to receive one or more additional inputs and the therapy response score may be further based on the one or more additional inputs, wherein the one or more additional inputs comprises at least one of user feedback or an environmental signal.
6. The system of clause 1 or of any of the preceding clauses, wherein the one or more physiological-based inputs comprises at least one of an activity or accelerometer signal, a cardiac-derived metric, a sleep score, a pain score, CMAP, EMG, respiration, an eCAP signal, posture, and/or other autonomic measures including organ sensing respiration.
7. The system of clause 1 or of any of the preceding clauses, wherein the one or more physiological-based inputs are received from at least one of a wearable device, a user device, or a neuromodulation therapy system.
8. The system of clause 1 or of any of the preceding clauses, wherein the one or more physiological-based inputs are received over a period of time and comprises one or more cardiac signals, and wherein the period of time is determined based on the one or more cardiac signals and input about the user's state.
9. The system of clause 1 or of any of the preceding clauses, wherein the memory stores further data for processing by the processor that, when processed, causes the processor to map the one or more changed physiological-based inputs to one or more events.
10. The system of clause 9, wherein the memory stores further data for processing by the processor that, when processed, causes the processor to generate a notification when at least one of the one or more events or the one or more physiological-based inputs are determined to be a risk to the user.
11. A system for tracking one or more physiological-based inputs of a user, the system comprising:
   a processor; and
   a memory storing data for processing by the processor, the data, when processed, causes the processor to:
      receive one or more physiological-based inputs at a first time,
      track the one or more physiological-based inputs after the first time and over a period of time to yield one or more changed physiological-based inputs, and
      determine one or more physiological responses based on a difference between the one or more physiological-based inputs received at the first time and the one or more changed physiological-based inputs; and
      determine if a physiological event has occurred based on the one or more physiological responses.
12. The system of clause 11, wherein the physiological event comprises a lead migration.
13. The system of clause 11 or of any of clauses 11-12, further comprising:
   a pulse generator configured to generate an electrical signal; and
   a lead in communication with the pulse generator and configured to transmit the electrical signal to an anatomical element.
14. The system of clause 11 or of clause 12 or of any of clauses 11-13, wherein the tracking the one or more physiological-based inputs are tracked with at least one of stimulation or without stimulation.
15. The system of clause 11 or of any of clauses 11-14, wherein the tracking is repeated over two or more days for a similar period of time each day.
16. The system of clause 11 or of any of clauses 11-15, wherein the period of time comprises a first period of time and a second period of time
17. A system for adjusting a neuromodulation therapy of a user, the system comprising:
   a processor; and
   a memory storing data for processing by the processor, the data, when processed, causes the processor to:
      track one or more physiological-based inputs at a first time;
      track the one or more physiological-based inputs after the first time and over a period of time to yield one or more changed physiological-based inputs; and
      adjust one or more parameters of the neuromodulation therapy based on a difference between the one or more physiological-based inputs and the one or more changed physiological-based inputs.
18. The system of clause 17, wherein the period of time comprises a first period of time and a second period of time, the first period of time corresponding to when the user is awake and the second period of time corresponding to when the user is asleep.
19. The system of clause 17, wherein the period of time comprises a first period of time and a second period of time, the first period of time corresponding to when the user is active and the second period of time corresponding to when the user is inactive.
20. The system of clause 17, wherein the one or more physiological-based inputs comprises one or more cardiac signals, and wherein the period of time is determined based on the one or more cardiac signals and input about the user's state.

## Claims

1. A system for tracking one or more physiological-based inputs of a user, the system comprising:
a processor; and
a memory storing data for processing by the processor, the data, when processed, causes the processor to:
receive one or more physiological-based inputs at a first time,
track the one or more physiological-based inputs after the first time and over a period of time to yield one or more changed physiological-based inputs, and
determine one or more physiological responses based on a difference between the one or more physiological-based inputs received at the first time and the one or more changed physiological-based inputs; and
determine if a physiological event has occurred based on the one or more physiological responses.

2. The system of claim 1, wherein the physiological event comprises a lead migration.

3. The system of any of the preceding claims, further comprising:
a pulse generator configured to generate an electrical signal; and
a lead in communication with the pulse generator and configured to transmit the electrical signal to an anatomical element.

4. The system of claim 3, wherein the pulse generator is part of a neuromodulation therapy system (100) and the electrical signals are configured for neuromodulation therapy.

5. The system of claim 3 or 4, wherein the tracking the one or more physiological-based inputs are tracked with at least one of stimulation or without stimulation.

6. The system of any of the preceding claims, wherein the tracking is repeated over two or more days for a similar period of time each day.

7. The system of any of the preceding claims, wherein the period of time comprises a first period of time and a second period of time.

8. The system of claim 7, wherein the first period of time corresponds to when the user is awake and the second period of time corresponding to when the user is asleep.

9. The system of claim 7, wherein the first period of time corresponds to when the user is active and the second period of time corresponding to when the user is inactive.

10. The system of any of the preceding claims, wherein the one or more physiological-based inputs comprises one or more cardiac signals.

11. The system of claim 10, wherein the period of time is determined based on the one or more cardiac signals and input about the user's state.

12. The system of any of the preceding claims, wherein the system further comprises a neuromodulation therapy system (100) configured to deliver electrical neuromodulation therapy to a user, wherein the data, when processed, further cause the processor to:
adjust one or more parameters of the neuromodulation therapy based on a difference between the one or more physiological-based inputs and the one or more changed physiological-based inputs.
